# EUROPEAN PATENT SPECIFICATION

(11) **EP 2 777 544 B1**
(45) Date of publication and mention of the grant of the patent: **24.04.2019**
(21) Application number: 14159102.4
(22) Date of filing: 12.03.2014
(51) Int. Cl.: A61F 2/82, A61B 17/12

(54) **Improved modifiable occlusion device**
Verbesserte modifizierte Okklusionsvorrichtung
Dispositif d'occlusion modifiable amélioré

(30) Priority: 13.03.2013 US 201313798818
(43) Date of publication of application: 17.09.2014
(73) Proprietor: DePuy Synthes Products, LLC, Raynham, MA 02767 (US)
(72) Inventor: Johnson, Kirk, Raynham, MA Massachusetts 02767 (US); Lorenzo, Juan A., Raynham, MA Massachusetts 02767 (US); Slazas, Robert, Raynham, MA Massachusetts 02767 (US)
(74) Representative: Carpmaels & Ransford LLP

(56) References cited:
- EP-A1- 1 652 494
- EP-A1- 2 505 149
- EP-A1- 2 777 543

## Description

### CROSS-REFERENCE TO RELATED APPLICATION

This application claims priority to U.S. Application No. 13/076,474, filed on March 31, 2011.

### BACKGROUND OF THE INVENTION

### 1. Field of the Invention

The invention relates to implants within body vessels and more particularly to occlusive devices including stents which are irreversibly modified based on localized pressure differentials.

### 2. Description of the Related Art

Vascular disorders and defects such as aneurysms and other arterio-venous malformations are especially difficult to treat when located near critical tissues or where ready access to a malformation is not available. Both difficulty factors apply especially to cranial aneurysms. Due to the sensitive brain tissue surrounding cranial blood vessels and the restricted access, it is very challenging and often risky to surgically treat defects of the cranial vasculature.

In the treatment of aneurysms by endovascular methods, the goal is to exclude the internal volume of the aneurysm sac from arterial blood pressure and flow. As long as the interior walls of the aneurysm are subjected to blood pressure and/or flow, there is a risk of the aneurysm rupturing.

Non-surgical treatments include vascular occlusion devices such as embolic coils deployed using catheter delivery systems. In a currently preferred procedure to treat a cranial aneurysm, the distal end of an embolic coil delivery catheter is initially inserted into non-cranial vasculature of a patient, typically through a femoral artery in the groin, and guided to a predetermined delivery site within the cranium. The aneurysm sac is then filled with embolic material that forms a solid, thrombotic mass that protect the walls from blood pressure and flow.

One inherent drawback to embolic treatments is that the aneurysm volume is permanently maintained due to the solid embolic mass implanted within them. Even after the aneurysm walls have been relieved of blood pressure and flow impingement, the walls cannot fully heal, reshape to a less distended formation, or be reincorporated back into the parent vessel wall. Also, if the size of the aneurysm created any "mass effect" type injury to the brain, the implanted embolic mass does not allow the aneurysm to shrink significantly after treatment.

When using a neck-occlusive approach to treat an aneurysm, the entrance or "neck" of the aneurysm is treated instead of the aneurysm volume itself. If the transfer of blood across the neck can be minimized, then stasis of the blood in the aneurysm volume can lead to formation of a natural thrombotic mass without the implantation of embolic materials. A natural thrombotic mass is preferable because it allows for an increased level of healing, including reduced distension of the aneurysm walls, and perhaps possible reincorporation of the aneurysm into the original parent vessel shape along the plane of the aneurysm's neck. The neck plane is an imaginary surface where the intima of the parent artery would be if not for formation of the aneurysm.

A significant challenge for many current neck-occlusive techniques is to substantially block the aneurysm neck in the parent vessel and yet not impede flow into perforator-type blood vessels, also referred to as small branch vessels, which branch off of the parent vessel, are very small in diameter, are numerous in some anatomical locations, and yet feed clinically important regions, especially within the brain. One example is the basilar artery, which has many perforator vessels feeding the pons and upper brain stem from the parent basilar artery. The use of a non-discriminatory neck occlusive device in this type of artery can unintentionally cause severe damage to the patient if the openings, known as "ostia", of the perforator vessels are blocked.

A typical basic configuration of neck-occlusive devices is a tubular, stent-like structure. These structures can be woven or wound from various fibers, laser-cut from metal, or made in various other ways. Many have interior struts or scaffolds. What most have in common is radial symmetry, meaning that they do not cover one portion, side or radial sector of the artery more or less porously than other sectors. Their symmetric construction, and therefore coverage of artery walls, is relatively homogeneous around any given transverse slice or cross-section, except where an interior strut may further reduce porosity from a micro-level perspective.

Several embodiments of an endoluminal vascular prosthesis are described in U.S. Patent No. 6,187,036 by Shaolian et al., for example, including one embodiment having fixed perfusion ports that can be aligned with diverging arteries. This prosthesis requires careful alignment of the perfusion ports with the adjacent vessels.

One example of an occlusion device directed to sealing an aneurysm while permitting flow to adjacent vessels is disclosed in U.S. Patent No. 7,156,871 by Jones et al. An expandable stent has a covering that is normally dissolvable in blood but, upon being locally activated by an activating agent, resists dissolution where activated. This device requires precise delivery of the separate activating agent.

Another type of aneurysm occlusion system is described by Bose et al. in U.S. Patent Publication No. 2007/0239261 having a plurality of pre-formed gaps or pores which allegedly expand in response to a fluid pressure differential at a side branch vessel. Various possibilities are mentioned including deflection of bendable elements such as small paddles, elastic stretching of pores, and defeating of surface tension by increased pressure differential.

It is therefore desirable to have a device which effectively occludes a neck of an aneurysm or other arterio-venous malformation in a parent vessel without blocking flow into perforator vessels communicating with the parent vessel.

EP2505149 discloses an occlusive device according to the preamble of claim 1 herinafter.

### SUMMARY OF THE INVENTION

An object of the present invention is to provide an occlusion device which substantially blocks flow into an aneurysm in a parent vessel yet quickly adapts to a pressure differential at an ostium of a perforator vessel to allow penetrating flow into the perforator vessel.

Another object of the present invention is to provide an occlusion device which is sensitive to a differentiating characteristic between the neck of the aneurysm and the ostium of a perforator vessel.
This invention results from the realization that the neck of an aneurysm in a parent vessel can be occluded without also occluding nearby vessels, such as perforator vessels, communicating with the parent vessel by providing a device having frangible material, associated with pores, which irreversibly erodes or ruptures, including deforming, substantially only based on differential pressure and penetrating fluid flow into the perforator vessels. The device effectively senses the presence of an ostium of a perforator vessel and modifies itself to
permit flow into the ostium through one or more of the pores, thereby minimizing ischemia, while continuing to substantially block flow into the aneurysm.

This invention features an occlusive device suitable for endovascular treatment of an aneurysm in a region of a parent vessel in a patient, as claimed hereinafter.

### BRIEF DESCRIPTION OF THE DRAWINGS

In what follows, preferred embodiments of the invention are explained in more detail with reference to the drawings and photographs, in which:
FIG. 1 is a schematic side view of an occlusive example device having a film overlying a support and positioned in a parent vessel below an aneurysm and above a perforator vessel;
FIG. 2 is a similar schematic side view of another example occlusive device having electro-spun fibers overlying a support;
FIG. 3 is a similar schematic side view of an example occlusive device having an erodible porous structure covering a support;
FIG. 4A is an enlarged schematic perspective, partial cross-sectional view of a portion of an alternative to the device shown in FIG. 3 having a durable porous structure;
FIG. 4B is a view of the durable porous structure of FIG. 4A after it has been impregnated with a selectively dissolving filler material;
FIG. 5 is a schematic side view of an occlusive device according to the present invention having a structure defining a plurality of pore features and positioned in a parent vessel below an aneurysm and above two perforator vessels;
FIG. 6 is a schematic cross-sectional view of an example pore feature including a frangible film-type substance;
FIG. 7A is a schematic cross-sectional view of an example pore feature including a degradable foam in one construction and, in another construction, illustrates initial rupture or erosion of the film of FIG. 6;
FIG. 7B is a view similar to FIG. 7A showing additional erosion within the pore feature; and
FIGS. 8A and 8B are schematic cross-sectional views of a pore feature according to the invention having an off-set, non-symmetrical frangible substance showing different amounts of erosion.

### DETAILED DESCRIPTION OF THE PRESENTLY PREFERRED EMBODIMENTS

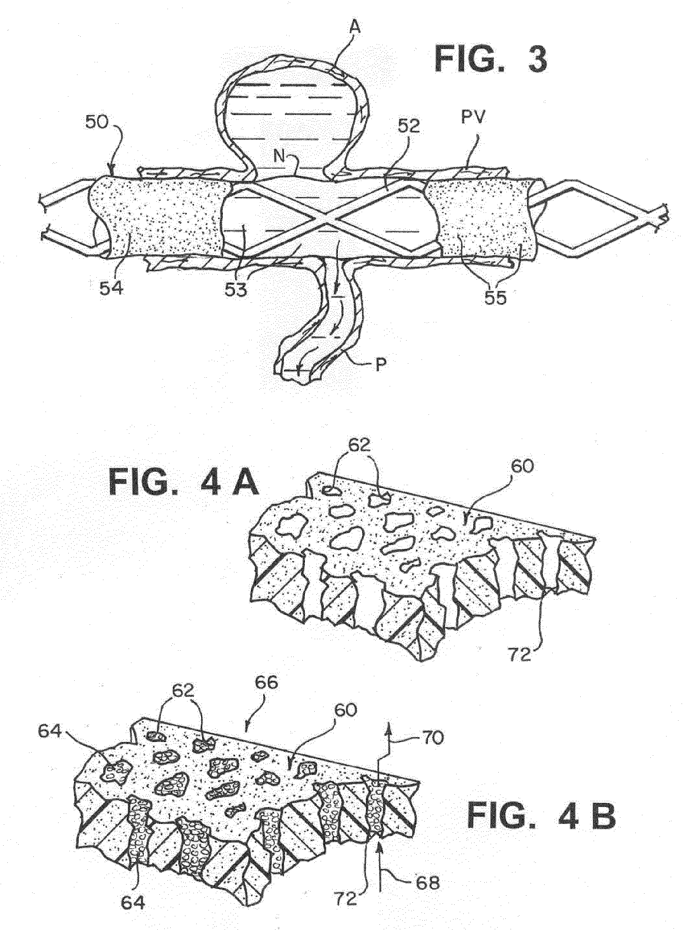

This invention may be accomplished by an occlusive device suitable for endovascular treatment of an aneurysm in a region of a parent vessel in a patient, with at least one type of supporting structure, such as metallic struts or porous foam, and at least one type of frangible material associated with pore features defined by the structure. The structure has dimensions suitable for insertion into vasculature of the patient to reach the region of the aneurysm in the parent vessel. The frangible material initially provides a substantial barrier to flow through the frangible material and is capable of at least one of localized rupturing and localized eroding, in the presence of a pressure differential arising at an ostium of a perforator vessel communicating with the parent vessel, within an acute time period to minimize ischemia downstream of the perforator vessel. Currently preferred constructions of devices are described below in relation to FIGS. 3-8B.

When considering the arterial system as a non-compressible fluid piping system, the aneurysm is a dead leg which does not drain by connecting to the low-pressure, venous side of the piping system. Over short time horizons, without considering growth or contraction of the aneurysm volume, any fluid volume that transfers across the neck plane must displace an equal amount of fluid volume from the aneurysm back into the parent vessel. The result is a net-zero transference across the neck plane for the aneurysm.

A perforator vessel differs from an aneurysm since the perforator vessel does drain directly or indirectly into the low pressure side of the piping system. There is a net-positive transference across the ostial plane because a given amount of fluid volume that crosses its ostial plane, that is, enters the perforator vessel through its ostium, is lost from the high pressure side of the system and does not force an equal amount back into the parent vessel as the aneurysm does.

In such a non-compressible fluid system, a net-zero transference across the neck plane causes a zero differential pressure across the neck plane. By comparison, a net-positive transference across the ostial plane can be detected by a positive differential pressure across the ostial plane. Therefore, differential pressure is a characteristic which a device can use to distinguish between the neck of an aneurysm and the ostia of perforator vessels. Since stent-like neck occlusion devices cover both a neck plane and an ostial plane in the same manner, the inventors have recognized that neck occlusion devices are needed that change their flow-impeding properties according to the presence of differential pressure across their walls, from interior to exterior.

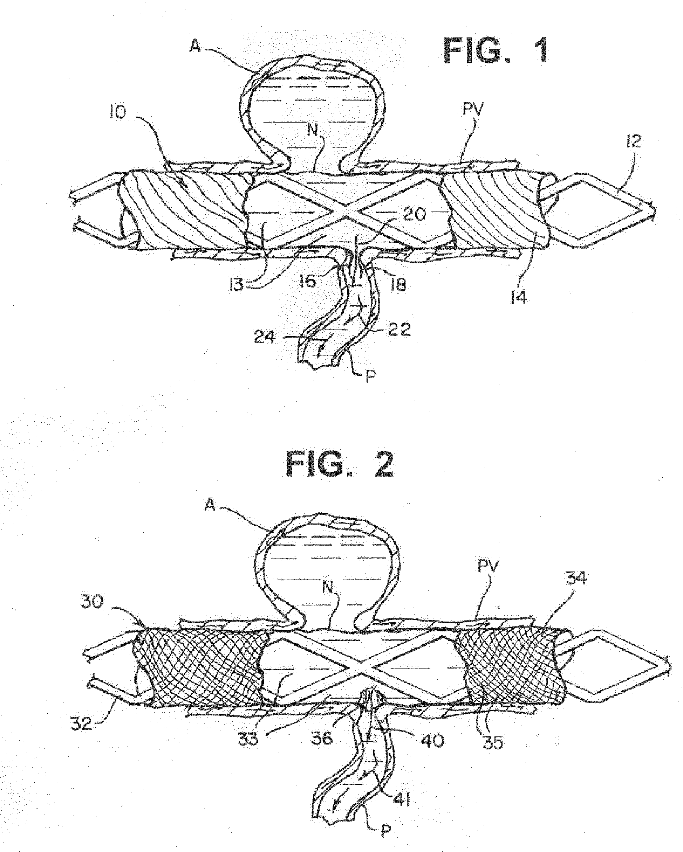

FIG. 1 schematically illustrates a novel tubular, stent-like device 10 implanted in a parent vessel PV with an upper aneurysm A and a lower perforator vessel P. Device 10 is substantially tubular and has structure such as metallic struts 12 defining relatively large openings 13 and supporting a frangible cover material 14 which includes a film-like substance that is capable of rupturing wherever a preselected differential pressure is achieved. Frangible material 14 is shown intact along the entire exterior of struts 12, including across aneurysm neck N, except where ruptured by differential pressure with resulting film flaps 16 and 18 slightly extending into the ostium of perforator vessel P. Penetrating fluid flow from parent vessel PV into perforator vessel P is illustrated by arrows 20, 22 and 24.

The frangible cover material 14 disrupts flow which would otherwise occur into aneurysm A and thereby enables a thrombus to form within aneurysm A. At the same time, frangible cover material 14 also enables blood to flow into perforator vessel P to continue feeding downstream tissues supplied by that vessel to minimize ischemia within those downstream tissues. Preferably, frangible cover material 14 provides a flow barrier at neck N for at least eight-to-twelve weeks to allow endothelial growth over device 10.

Device 10 can be either self-expanding or balloon expanded, with supporting scaffold-like structure 12 made by any of several typical stent fabrication methods. The struts 12 themselves are solid, typically metal, and do not change behavior according to the distinguishing feature of differential pressure across either an aneurysm neck or the ostium of a branching vessel. In the preferred embodiment, the struts 12 serve as a self-expanding scaffold made by laser-cutting a pattern of struts into a nitinol (NiTi) tube. The primary purposes of this structural component are to facilitate delivery of a film or other frangible cover material 14 to the target vessel, and to hold cover material 14 in apposition to the vessel wall once deployed. If the covering 14 is structurally sufficient to enable delivery and to hold position in the artery on its own, this scaffold 12 may not be needed.

The open areas 13 within the scaffold 12 are subsequently covered by a film 14 which does respond according to the level of differential pressure felt across its wall thickness. There is a net positive differential pressure across a branching vessel's ostium and none across the neck of an aneurysm, typically ranging from one to fifty mm Hg. This film 14 can be made from any number of substances, as long as it has the minimum characteristics of biocompatibility and frangibility in the presence of a preselected, sufficient differential pressure. Suitable biocompatible compositions for frangible material 14 include films or matrices of cellulose, alginate, cross-linked gels, and very thin polymer films of materials such as urethane and/or poly-glycolic acid. The film 14 need not be erodible or bioabsorbable since it is the action of rupture in the presence of sufficient differential pressure that creates the permanent, localized modification of increased flow across its wall-thickness. Similarly, although microscopic pores or other openings could be formed in the film 14 having average diameters such as described for other embodiments below, it is acceptable for the film 14 to be a continuous sheet of material because the action of rupture increases flow where needed, as sensed by sufficient differential pressure to cause the rupture.

The thickness of the film layer is determined by its desired rupture strength, but should not occupy a significant amount of cross-sectional area in the artery in order to minimize interference with normal fluid flow through the parent vessel. Less than five percent area occupation is desired. The thickness of the film is selected to achieve a desired frangibility at a minimum differential pressure within an acute time period to minimize ischemia downstream of the perforator vessel. In some constructions, the acute time period is preferably within a period of less than ten minutes, more preferably less than five minutes, in a majority of patients under typical conditions, that is, not including hypothermic or artificially depressed blood pressure conditions. The rupture strength should be adjusted so that the film is strong enough to survive delivery and placement within the target artery, but weak enough to rupture in the presence of the persistent, net-positive differential pressure across the ostium of small branching vessels. Desirable rupture strengths are expected to be in the range of 1 to 50 mmHg differential pressure.

An alternative tubular device 30, FIG. 2, has struts 32 which are similar to struts 12, FIG. 1, and define relatively large openings 33, FIG. 2. Device 30 further includes frangible material 34 which is formed from very thin fibers 35 in this construction that establish a porous mesh or matte outer layer. Frangible material 34 has a density sufficient to disrupt normal fluid flow at neck N to create stasis within aneurysm A to enable thrombi to form therein, yet a sufficient number of the fibers 35 part or separate to form opening 36 at the ostium of perforator vessel P when a threshold pressure differential is exceeded to enable blood to flow as illustrated by arrows 40 and 41.

In a preferred construction, these fibers 35 are applied via "electro-spinning", where a liquefied polymer such as polyvinylidene fluoride (PVDF) exiting a dispenser tip has a voltage applied to it, producing a very fine strand having an average strand thickness or diameter of one nanometer up to about ten microns. A number of controls over the construction of the fiber layer can be manipulated, such as the thickness of individual strands, the total number of strands applied, the angle at which the strand lays on the tubular scaffold, and the angles between strands which cross each other. Various electro-spinning techniques can be utilized, such as those described by Norton in U.S. Patent No. 2,048,651. Other electro-spinning techniques are described by Cooley in U.S. Patent No. 692,631, by Morton in U.S. Patent No. 705,691, and by Formhals in U.S. Patent Nos. 1,975,504 and 2,349,950 for example. The resulting characteristics of the fiber layer as manufactured, before implantation, include percentage area covered, average pore or opening size, total wall thickness, and hydraulic permeability, which provides a gross measurement of the volumetric flow rate of a certain liquid across the layer, in this case blood. In some constructions, the overall layer thickness of material 34 is about 10 microns to about 500 microns, more preferably 30 microns to 200 microns. The average opening diameter between fibers, as measured from scanning electron microscope images along a plane substantially parallel to the surface of material 35, is preferably at least 10 microns before implantation in a patient. Average openings of about 10 microns permit a small quantity of whole blood, including red blood cells, to pass through the sidewalls of device 30 to provide some nourishment to surrounding tissues, while initially providing a substantial barrier to flow through material 34. As one or more fibers rupture in the presence of sufficient differential pressure such
as at the ostium of the perforator vessel P, opening 36 is preferably formed to be from 50 to 500 microns, more typically 100 to 300 microns in diameter.

The mechanism by which a sufficient number of these fibers "part" or separate in the presence of sufficient differential pressure is primarily that individual fibers will break, that is, rupture, in the localized areas of higher fluid flow. In alternate constructions, a mixture of biologically durable and degradable materials are utilized for the fibers. In regions of the fiber mesh that cover the ostium of a branching vessel, the local differential pressure is net positive and causes a persistent flow through the wall thickness of the layer. These broken fibers in the region of the layer covering the ostium of a branching vessel serve to increase the blood flow to that branching vessel preferentially compared to the region covering the aneurysm neck. The controllable factors in the construction of the frangible fiber layer 34, FIG. 2, should be adjusted such that the fibers 35 break in areas with differential pressure preselected to be a threshold rupture pressure between 1 and 50 mmHg. The thickness of the fiber layer is determined by its rupture strength, but should not occupy a significant amount of cross-sectional area in the artery. Less than five percent area occupation is desired. In some constructions, a sufficient number of fibers break or erode within an acute time period, to minimize ischemia downstream of the perforator vessel, that is preferably within a period of less than ten minutes, more preferably less than five minutes, in a majority of patients under typical conditions, that is, not including hypothermic or artificially depressed blood pressure conditions.

Tubular device 50, FIG. 3, is an example constructed with struts 52 arranged as a scaffold to define open areas or cells 53. This scaffold 52 can be either self-expanding or balloon expanded, made by any of several typical fabrication methods. The scaffold 52 is then covered with a layer 54 that has very fine pores 55 and allows a limited amount of flow across its wall thickness in the presence of a net positive differential pressure. This layer 54 can be constructed by many methods, for example foaming, lyophilization, gaseous extraction, etching, firing, or deposition. The material of layer 54 can be any biocompatible material that is subject to erosion due to fluid flow and/or erosion due to bioabsorption including consumption by live cells. In the preferred embodiment, polycaprolactone (PCL) is deposited in a somewhat sparse matrix such that it is porous as a bulk material. Other potential materials include polylactic acid (PLA), polyglycolic acid (PGA), polysaccharides, colloidal compounds, and some lipid products.

In an alternate configuration as shown in FIGS. 4A and 4B, a structure 60 of a durable, non-erodible, non-bioabsorbable material is first constructed. This flexible, elastic structure, such as a solidified urethane foam or expanded polytetrafluoroethylene (PTFE), has relatively large pores 62 so that structure 60, by itself, covers too little of the open area, has too large an average pore size, and has a hydraulic permeability that is too great to sufficiently impede or restrict flow into an aneurysm. In other words, structure 60, which may be reinforced with metal struts, establishes a maximum porosity for a device according to the present invention. Although pores 62 are shown in cross-section with relatively straight passages, such as passage 72, for simplicity of illustration, in many constructions the passages are more complex and convoluted. Pores 62 are preferably formed to be from 50 to 500 microns in average diameter, more typically 100 to 300 microns in average diameter, as measured from scanning electron microscope images along a plane substantially parallel to the surface of structure material 60.

After fabricating the structure 60, a second substance 64 that is erodible is interstitially combined with the structure 60 to form a device 66, FIG. 4B. The second material 64, such as PCL or other materials listed above, preferably is deposited as particles or a microporous foam such that the material 64 has a desired level of porosity itself, that is, it is not an impermeable bulk material. In certain constructions, material 64 defines openings having an average diameter of preferably at least 10 microns before implantation in a patient. Average openings of about 10 microns permit a small quantity of whole blood, including red blood cells, to pass through the sidewalls of device 66, as indicated by internal flow arrow 68 entering into passage 72 and external flow arrow 70 emerging from passage 72, to provide some nourishment to surrounding tissues, while initially providing a substantial barrier to flow through device 66. In the areas of net positive differential pressure, over the ostia of branching vessels, the persistent, penetrating flow through the wall of the combined layer will cause the second material 64 to respond by preferentially eroding, typically including biodegrading, more rapidly in one or more pores 62. The first purpose of the structure material 60 is to impose an upper limit on the increase in porosity, and therefore flow, to that of the structure 60 itself after all of the second material 64 has been removed. Its second purpose is to intensify the erosion, typically including biodegradation, of the second material 64 by concentrating the differential pressure provided by the branching vessel into a smaller porous area. This will improve the preferential nature by which the combined layer of device 66 will erode above branching vessels more quickly than in the general body of the device, including above an aneurysm neck.

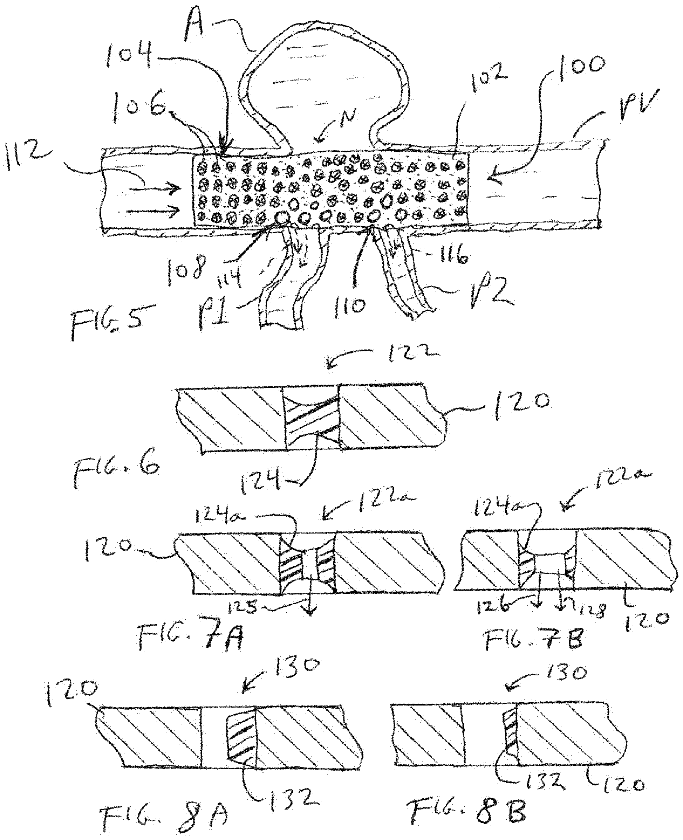

Tubular device 100, FIG. 5, has a durable, preferably flexible structure 102 defining a plurality of pore features 104 and is shown positioned in a parent vessel PV below an aneurysm A, having a neck N, and above two perforator vessels P1 and P2. Preferably, device 100 can be inserted through parent vessel PV in a collapsed condition and then expanded, by self-expansion or by balloon expansion, when device 100 straddles the aneurysm neck N. Pore features 104, represented by circles in this construction, have a pre-established porosity. At least one frangible material 106 is associated with the pore features 104 as represented by the "x" in each pore feature 104, except for several substantially open pore features 108 and 110, where blood flow 112 has caused the pore features 108 and 110 to allow some flow, dashed arrows 114 and 116, into perforators P1 and P2, respectively. In other words, frangible material 106 generates a first condition for the pore features 104 which initially provides a substantial barrier to flow through the frangible material 106, such as at neck region N, and, for at least a majority of the pore features 104, is capable of at least one of localized rupturing and localized eroding, in the presence of a localized pressure differential arising at an ostium of a perforator vessel P1 and/or P2 communicating with the parent vessel PV to generate, within an acute time period, a second condition for pore features 108, 110 experiencing the localized pressure differential to minimize ischemia downstream of the perforator vessels by allowing sufficient blood to flow into the perforator vessels to feed downstream tissue territories of those vessels.

In a number of constructions, at least some of the pore features have geometries that differ from the geometries of other of the pore features. Various geometries include circles, ellipsoids, and trapezoids. The geometric size of the pores is substantially constant along the length of the structure 102 in some constructions and, in other constructions, varies along the length. The number of pores is substantially uniform along the length of the structure 102 in some constructions and, in other constructions, varies along the length.

FIG. 6 is a schematic cross-sectional view of structure 120 defining an example pore feature 120 including a frangible film-type substance 124 in a first condition. Substance 124 ruptures in the presence of a sufficient differential pressure as found at or near a perforator vessel, but not at an aneurysm neck. Substance 124 is bio-absorbable or biodegradable in some constructions.

FIG. 7A is a schematic cross-sectional view of an example pore feature 122a including a degradable foam 124a in one construction and, in another construction, illustrates initial rupture or erosion of the film 124 of FIG. 6. In other words, FIG. 7A represents a first condition in the first construction, with minimal fluid flow 125 through pore feature 122a, and represents a second condition for FIG. 6 in the other construction. Preferably, the initial porosity of pore feature 122a allows a minimal amount of flow through its wall thickness to the ostia of perforator vessels but none to an aneurysm neck. Then, over a period of time, the material forming the pores subjected to the differential pressure (and therefore blood flow) will eventually erode and become larger, allowing increased blood flow. The time period and material's resistance to erosion preferably is sufficiently high so as not to erode due to very small transferences across the aneurysm neck before stasis is established and a thrombus is formed in the aneurysm. However, it is preferable for the time period and resistance to erosion to be sufficiently low that the persistent differential pressure of a perforator vessel eventually erodes the material away in the vicinity of the ostium.

FIG. 7B is a view similar to FIG. 7A showing additional erosion within the pore feature 122a to allow greater flow, as represented by arrows 126 and 128. In other words, where FIG. 7A represents a first condition, FIG. 7B represents a second condition; where FIG. 7A represents the beginning of a second condition for the device of FIG. 6, then FIG. 7B represents an increased porosity and increased blood flow 126, 128 to a perforator vessel in the second condition.

The initial porosity of the frangible substance is controlled in some constructions by the geometry of the substance within the pores and, in other constructions, is primarily controlled by material absorption rate. FIGS. 8A and 8B are schematic cross-sectional views of a pore feature 130, according to an embodiment of the invention and having an off-set, non-symmetrical frangible substance 132 showing different amounts of erosion over time. Preferably, pore feature 130, FIG. 8A, allows a minimal amount of flow to a perforator vessel immediately after implantation but limits flow into an aneurysm neck, both mechanically and because of a net-zero pressure transference across the neck plane. Then, over time, the material subject to differential pressure (and therefore blood flow) will eventually degrade and become larger, allowing increased blood flow. The time period and material's resistance to erosion preferably is sufficiently high so as not to erode due to very small transferences across the aneurysm neck before stasis is established and a thrombus is formed in
the aneurysm. However, it is preferable for the time period and resistance to erosion to be sufficiently low that the persistent differential pressure of a perforator vessel eventually erodes the material away in the vicinity of the ostium.

In one technique of manufacture, structure 120 is oriented vertically so that material 132 accumulates substantially on one side of the pore features 130. In other manufacturing techniques, a channel or other opening in a majority of pores is created by laser, water jet, or other penetration technique.

Thus, while there have been shown, described, and pointed out fundamental novel features of the invention as applied to a preferred embodiment thereof, it will be understood that various omissions, substitutions, and changes in the form and details of the devices illustrated, and in their operation, may be made by those skilled in the art without departing from the scope of the invention. For example, it is expressly intended that all combinations of those elements and/or steps that perform substantially the same function, in substantially the same way, to achieve the same results be within the scope of the invention. Substitutions of elements from one described embodiment to another are also fully intended and contemplated. It is also to be understood that the drawings are not necessarily drawn to scale, but that they are merely conceptual in nature. It is the intention, therefore, to be limited only as indicated by the scope of the claims appended hereto.

## Claims

1. An occlusive device (100) suitable for endovascular treatment of an aneurysm in a region of a parent vessel in a patient, comprising:
a structure (102) having pre-established pore features (104) and having dimensions suitable for insertion into vasculature of the patient to reach the region of the aneurysm in the parent vessel; and
a frangible material (106) within the pore features (104) to generate:
a first condition for the pore features (104) which initially provides a substantial barrier to flow through the frangible material and, for at least a majority of the pore features (104), is capable of at least one of localized rupturing and localized eroding, in the presence of a localized pressure differential arising at an ostium of a perforator vessel communicating with the parent vessel to generate, within an acute time period,
a second condition for pore features experiencing the localized pressure differential to minimize ischemia downstream of the perforator vessel and allow increased blood flow,
**characterised in that** in each of the first and second conditions the frangible material (106) within the pore features (104) takes the form of a frangible substance (132) being off-set to one side of each of the respective pore features.

2. The occlusive device (100) of claim 1 wherein at least some of the pore features have geometries that differ from the geometries of other of the pore features.

3. The occlusive device of claim 1 wherein the pore features are regularly spaced along the length of the structure.

## Patentansprüche

1. Okklusionsvorrichtung (100), die für endovaskuläre Behandlung eines Aneurysmas in einem Bereich eines Trägergefäßes in einem Patienten geeignet ist, umfassend:
eine Struktur (102) mit vorab eingerichteten Porenmerkmalen (104) und Abmessungen, die für das Einführen in das Gefäßsystem des Patienten geeignet sind, um den Bereich des Aneurysmas im Trägergefäß zu erreichen, und
ein bröckeliges Material (106) mit den Porenmerkmalen (104), um Folgendes zu erzeugen:
einen ersten Zustand für die Porenmerkmale (104), der anfänglich eine wesentliche Barriere für das Durchfließen des bröckeligen Materials bereitstellt und für mindestens eine Mehrheit der Porenmerkmale (104) zu örtlichem Brechen und/oder örtlichem Erodieren bei Vorliegen einer örtlichen Druckdifferenz in der Lage ist, die an einem Ostium eines Perforansgefäßes vorherrscht, das mit dem Trägergefäß kommuniziert, um innerhalb eines akuten Zeitraums einen zweiten Zustand für Porenmerkmale, die der örtlichen Druckdifferenz ausgesetzt sind, zu erzeugen, um stromabwärts vom Perforansgefäß Ischämie zu minimieren und erhöhte Durchblutung zu gestatten, **dadurch gekennzeichnet, dass** in jedem des ersten und zweiten Zustands das bröckelige Material (106) innerhalb der Porenmerkmale (104) die Form einer bröckeligen Substanz (132) annimmt, die von jedem der jeweiligen Porenmerkmale zu einer Seite hin versetzt ist.

2. Okklusionsvorrichtung (100) nach Anspruch 1, wobei mindestens einige der Porenmerkmale Geometrien haben, die sich von den Geometrien anderer der Porenmerkmale unterscheiden.

3. Okklusionsvorrichtung nach Anspruch 1, wobei die Porenmerkmale regelmäßig entlang der Länge der Struktur beabstandet sind.

## Revendications

1. Dispositif d'occlusion (100) approprié pour un traitement endovasculaire d'un anévrisme dans la région d'un vaisseau parent d'un patient, comprenant :
une structure (102) ayant des éléments de pores préétablis (104) et ayant des dimensions appropriées pour l'insertion dans le système vasculaire du patient de manière à atteindre la région de l'anévrisme dans le vaisseau parent ; et
un matériau frangible (106) à l'intérieur des éléments de pores (104), pour générer :
un premier état pour les éléments de pores (104), qui fournit initialement une barrière majeure à l'écoulement à travers le matériau frangible, et, pour au moins une majorité des éléments de pores (104), est capable soit d'une rupture localisée soit d'une érosion localisée, en présence d'une différence de pression localisée survenant au niveau d'un ostium d'un vaisseau perforateur communiquant avec le vaisseau parent de manière à générer, pendant une période de temps extrêmement courte,
un deuxième état pour les éléments de pores subissant la différence de pression localisée de manière à minimiser l'ischémie en aval du vaisseau perforateur et à permettre un débit sanguin accru, **caractérisé en ce que** dans chacun parmi les premier et deuxième états, le matériau frangible (106) à l'intérieur des éléments de pores (104) prend la forme d'une substance frangible (132) décalée d'un côté de chacun des éléments de pores respectifs.

2. Dispositif d'occlusion (100) selon la revendication 1, dans lequel au moins certains des éléments de pores présentent des géométries qui sont différentes des géométries de certains autres des éléments de pores.

3. Dispositif d'occlusion selon la revendication 1, dans lequel les éléments de pores sont espacés régulièrement le long de la longueur de la structure.
